(19) Europäisches Patentamt
European Patent Office
Office européen des brevets

(11) **EP 3 219 260 B1**

(12) **EUROPÄISCHE PATENTSCHRIFT**

(45) Veröffentlichungstag und Bekanntmachung des Hinweises auf die Patenterteilung:
**22.05.2019 Patentblatt 2019/21**

(21) Anmeldenummer: **17161123.9**

(22) Anmeldetag: **15.03.2017**

(51) Int Cl.:
*A61B 6/00* (2006.01)      *G06T 11/00* (2006.01)
*A61B 6/03* (2006.01)      *A61B 6/12* (2006.01)
*G06T 7/11* (2017.01)

(54) **VORRICHTUNG UND VERFAHREN ZUM ABGRENZEN EINES METALLOBJEKTS FÜR EINE ARTEFAKTREDUKTION IN TOMOGRAPHIEBILDERN**

DEVICE AND METHOD FOR DELINEATING A METAL OBJECT FOR ARTEFACT REDUCTION IN TOMOGRAPHIC IMAGES

DISPOSITIF ET PROCÉDÉ DESTINÉS À LIMITER UN OBJET MÉTALLIQUE POUR UNE RÉDUCTION D'ARTEFACT DANS DES IMAGES TOMOGRAPHIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2016 DE 102016204226**

(43) Veröffentlichungstag der Anmeldung:
**20.09.2017 Patentblatt 2017/38**

(73) Patentinhaber: **Siemens Healthcare GmbH 91052 Erlangen (DE)**

(72) Erfinder: **Manhart, Michael 90762 Fürth (DE)**

(56) Entgegenhaltungen:
**DE-A1-102011 075 912      DE-B3-102013 218 819
US-A1- 2015 029 178**

- **MEHRANIAN ABOLFAZL ET AL: "3D Prior Image Constrained Projection Completion for X-ray CT Metal Artifact Reduction", IEEE TRANSACTIONS ON NUCLEAR SCIENCE, IEEE SERVICE CENTER, NEW YORK, NY, US, Bd. 60, Nr. 5, 2. Oktober 2013 (2013-10-02), Seiten 3318-3332, XP011528933, ISSN: 0018-9499, DOI: 10.1109/TNS.2013.2275919 [gefunden am 2013-10-09]**
- **JIN PENGCHONG ET AL: "Joint metal artifact reduction and segmentation of CT images using dictionary-based image prior and continuous-relaxed potts model", 2015 IEEE INTERNATIONAL CONFERENCE ON IMAGE PROCESSING (ICIP), IEEE, 27. September 2015 (2015-09-27), Seiten 798-802, XP032826531, DOI: 10.1109/ICIP.2015.7350909 [gefunden am 2015-12-09]**

EP 3 219 260 B1

**EP 3 219 260 B1**

**Beschreibung**

Die Erfindung betrifft ein Verfahren zum Abgrenzen eines Metallobjekts für eine Artefaktreduktion in Tomographiebildern. Zu der Erfindung gehört auch eine Vorrichtung zum Durchführen des erfindungsgemäßen Verfahrens.

Zum Erzeugen von Tomographiebildern werden Projektionsbilder eines Körpers verwendet, wie sie durch eine Projektionseinrichtung, beispielsweise einem Computertomographen (CT) erzeugt werden können. Der Körper kann ein menschlicher oder tierischer Körper oder auch ein Gegenstand sein. Auf der Grundlage der Projektionsbilder wird ein Volumenmodell zumindest eines Teils des Körpers gebildet. Eine Schnittansicht des Volumenmodells stellt jeweils ein Tomographiebild oder ein Tomogramm dar.

Befindet sich in dem Körper ein Metallobjekt, beispielsweise eine Metallklammer, so weist das Volumenmodell sogenannte Metallartefakte auf. Diese äußern sich darin, dass für einzelne Voxel (Volumenelemente) des Volumenmodells in unmittelbarer Umgebung des Metallobjekts nicht die Eigenschaft des dort befindlichen Körpergewebes beschreiben, sondern ebenfalls quasi wie bei einer Überbelichtung oder Unterbelichtung Voxelwerte aufweisen, die den Voxelwerten des Metallobjekts selbst sehr ähnlich sind und daher kaum von diesen zu unterscheiden sind.

Zur Reduktion solcher Metallartefakte ist beispielsweise ein Verfahren aus der wissenschaftlichen Veröffentlichung von Veldkamp et al. bekannt (W. J. H. Veldkamp, R. m. S. Joemai, A. J. van der Molen, J. Geleijns, "Development and validation of segmentation and interpolation techniques in sinograms for metal artifact suppression in CT", Medical Physics 37, 620 (2010), doi: 10.1118/1.3276777, http://dx.doi.org/10.1118/1.3276777).

Durch eine solche Methode oder auch durch manuelles Markieren des Metallobjekts in einem Tomographiebild kann die Form des Metallobjekts ermittelt und beim Berechnen eines Volumenmodells berücksichtigt werden. Hierdurch wird im Volumenmodell der Einfluss des Metallobjekts auf die Voxel in seiner Umgebung reduziert.

Aus der US 2015/029178 A1 ist bekannt, eine Maske für Metallartefakte zweistufig zunächst auf Grundlage eines Volumenmodells und anschließend auf der Grundlage daraus erzeugter Projektionsbilder zu bilden.

In einem Fachbeitrag von M. Abolfazl et al. (Mehranian Abolfazl et al., "3D Prior Image Constrained Projection Completion for X-ray CT Metal Artefact Reduction", IEEE Transactions on Nuclear Science, IEEE Service Center, New York, US, Bd. 60, Nr. 5, 1. Oktober 2013, Seiten 3318-3332, ISSN: 0018-9499, DOI: 10.1109/TNS.2013.2275919) ist beschrieben, ausgehend von einem Volumenmodell zunächst Projektionen zu erzeugen und diese dann mittels einer Maximum-A-Posteriori (MAP) Wahrscheinlichkeitsberechnung zu optimieren, um daraufhin mittels einer Rückprojektion ein Volumenmodell eines Metallartefakts und ein Volumenmodell eines metallfreien Körpers zu erhalten, die dann vereint werden.

Aus einem Fachbeitrag von Jin et al. (JIN, P. et al.: "Joint Metal Artifact Reduction and Segmentation of CT Images using Dictionary-Based Image Prior and Continuously-Relaxed Potts Model", in Proceedings of the IEEE International Conference on Image Processing 2015 (ICIP 2015), 798-802) ist ein Verfahren zum Bilden von Masken für Metallartefakte bekannt, das ohne die eigentlichen Projektionsdaten auskommt.

Das Dokument DE 10 2011 075 912 A1 beschreibt ein Verfahren, bei welchem von einem Körper zwei Volumenmodelle auf Grundlage unterschiedlicher Röntgen-Tomographieaufnahmen erzeugt werden. Durch Vereinen der beiden Volumenmodelle kann ein verbessertes Volumenmodell gebildet werden.

Der Erfindung liegt die Aufgabe zugrunde, für eine Artefaktreduktion eines Metallobjekts in Tomographiebildern eine Vorrichtung und ein Verfahren bereitzustellen, mittels welchen das Metallobjekt automatisiert abgegrenzt oder beschrieben werden kann.

Die Aufgabe wird durch die Gegenstände der unabhängigen Patentansprüche gelöst. Vorteilhafte Weiterbildungen der Erfindung sind durch die Merkmale der abhängigen Patentansprüche, die folgende Beschreibung sowie die Figuren gegeben.

Durch die Erfindung ist ein Verfahren zum Abgrenzen eines Metallobjekts bereitgestellt. Die Abgrenzung ermöglicht die Durchführung einer Artefaktreduktion in Tomographiebildern mit an sich bekannten Methoden. Es wird für eine solche Methode aber eine Beschreibung der dreidimensionalen Form des Metallobjekts benötigt, die nun durch das Verfahren in einer automatisierten Form bereitgestellt werden kann.

Das Verfahren geht davon aus, dass Projektionsbilder eines das Metallobjekt enthaltenen Körpers aus einer Projektionseinrichtung empfangen werden. Die Projektionseinrichtung kann beispielsweise ein Computertomograph sein, welcher die Projektionsbilder röntgenbasiert oder auf Basis einer Positronen-Emission erzeugt. Die Projektionseinrichtung kann beispielsweise eine Röntgen-C-Bogen-Anlage sein. Die Projektionsbilder enthalten Pixel (Bildelemente), die jeweils einen Pixelwert aufweisen, der beispielsweise eine Dämpfungseigenschaft des Körpers bezüglich der von der Projektionseinrichtung verwendeten Strahlung angeben. Die Einheit eines Pixelwerts kann beispielsweise HU (Hounsfield Unit) sein.

Auf der Grundlage der Projektionsbilder wird in an sich bekannter Weise ein Volumenmodell des Körpers gebildet, welches einzelne Voxel (Volumenelemente) aufweist, von denen jedes durch einen Voxelwert eine jeweilige Dämpfungseigenschaft des Voxels bezüglich der Strahlung der Projektionseinrichtung angibt. Eine bekannte Methode zum Erzeugen eines solchen Volumenmodells ist die gefilterte Rückprojektion.

In einem solchen Volumenmodell kann nun das Metallobjekt Metallartefakte, beispielsweise eine sternförmige Region um das eigentliche Metallobjekt herum, verursachen. Die Voxel dieser sternförmigen Region weisen nahezu dieselben Voxelwerte auf wie die Voxel des Metallobjekts selbst. Um dennoch die Voxel des Metallobjekts selbst erkennen zu können, sind folgende erfindungsgemäße Schritte vorgesehen.

In dem Volumenmodell werden auf der Grundlage eines Segmentierungskriteriums Voxel, die gemäß dem Segmentierungskriterium Teil des Metallobjekts und der Metallartefakte sind, ausgewählt und zu einer Volumenmaske kombiniert oder zusammengefasst. Das Segmentierungskriterium kann beispielsweise eine Schwellwertdetektion für die besagten Pixelwerte vorsehen oder verwenden. Mit anderen Worten werden all diejenigen Voxel dem Metallobjekt zugeordnet, deren Voxelwerte größer oder kleiner als ein vorbestimmter Schwellenwert sind. Diese Voxel bilden in ihrer Gesamtheit die Volumenmaske. In der Regel stellt eine solche Volumenmaske eine zusammenhängende Region dar. Diese erste Volumenmaske (später wird eine zweite Volumenmaske eingeführt) wird auf die Projektionsbilder projiziert. Diese Projektion stellt eine Projektionsmaske für das jeweilige Projektionsbild dar. Zum Projizieren der ersten Volumenmaske kann in an sich bekannter Weise die Methode der Vorwärtsprojektion verwendet werden. Diese stellt also den Vorgang der Projektion nach, wie sie auch durch die Projektionseinrichtung durchgeführt wird. Eine Projektionsmaske stellt also den Schatten der ersten Volumenmaske auf dem jeweiligen Projektionsbild dar.

Jede Projektionsmaske wird dann auf der Grundlage von Pixelwerten der jeweiligen Projektionsbilder angepasst. Dies ist möglich, da die Projektionsbilder selbst artefaktfrei sind, d.h. Pixelwerte von Pixeln, die benachbart zu der Projektion des Metallobjekts selbst sind, sind nicht von diesem beeinflusst. Zwar lässt sich in den Projektionsbildern das Metallobjekt selbst nicht so deutlich von z.B. Knochen unterscheiden wie im Volumenmodell. Die Lage des Metallobjekts ist aber durch die Projektionsmaske bekannt.

Aus den angepassten Projektionsmasken wird dann die besagte zweite Volumenmaske ermittelt. Dies kann beispielsweise auf der Grundlage der beschriebenen Methode der Rückprojektion durchgeführt werden. Die zweite Volumenmaske beschreibt dann in dem Volumen eine Region innerhalb der Metallartefakte und umgibt aber noch die Voxel des Metallobjekts selbst. Nun wird auf der Grundlage von Voxelwerten des Volumenmodells die zweite Volumenmaske angepasst. Dies ist nun möglich, da man zwischen den Voxelwerten des Metallobjekts selbst und den (sehr ähnlichen) Voxelwerten der Metallartefakte mittels der zweiten Volumenmaske unterscheiden kann. Hierzu kann man insbesondere zwischen Voxelwerten außerhalb der Volumenmaske einerseits, die aber immer noch innerhalb der Metallartefakte liegen, und Voxelwerten innerhalb der Volumenmaske unterscheiden. Die angepasste Volumenmaske wird dann als Beschreibung der dreidimensionalen Form des Metallobjekts bereitgestellt.

Beispielsweise kann nun auf der Grundlage der angepassten Volumenmaske erneut aus den Projektionsbildern ein finales Volumenmodell gebildet werden. Die angepasste Volumenmaske kann hierbei beispielsweise dazu genutzt werden, solche Pixelwerte von Pixeln der Projektionsbilder auszublenden oder zu ignorieren, von denen man anhand der angepassten Volumenmaske detektiert oder erkennt, dass sie das Metallobjekt darstellen. Aus dem finalen Volumenmodell können dann in der beschriebenen Weise die Tomographiebilder als Schnittdarstellungen gebildet werden.

Durch die Erfindung ergibt sich der Vorteil, dass man das Metallobjekt zunächst in dem ersten Volumenmodell detektiert, wo es sich durch seine Voxelwerte deutlich von Voxelwerten des Gewebes und Knochen des Körpers unterscheidet. Allerdings ist die genaue Form oder sind die Grenzen des Metallobjekts aufgrund der Metallartefakte im Volumenmodell verfälscht. Durch die Projektionsmasken in den Projektionsbildern kann dann anhand der artefaktfreien Pixelwerte die jeweilige Projektionsmaske verfeinert oder angepasst werden. Diese können dann zurück zu dem Volumen projiziert werden, wodurch die zweite Volumenmaske entsteht, die es dann ermöglicht, genauer zwischen den Voxelwerten des Metallobjekts selbst und den Voxelwerten des artefaktbehafteten Volumenhintergrunds (d.h. der Umgebung des Metallobjekts) zu unterscheiden. Hieraus ergibt sich dann in vorteilhafter Weise die angepasste Volumenmaske. Mit anderen Worten werden durch das Verfahren die Vorteile der Analyse im Volumenmodell (deutliches Metallobjekt) einerseits und der Projektionsbilder (artefaktfreie Abbildung) andererseits kombiniert.

Ein Aspekt der Erfindung betrifft das Anpassen der Projektionsmasken in den Projektionsbildern. Das Anpassen erfolgt gemäß dem Aspekt, indem in dem jeweiligen Projektionsbild eine die Projektionsmaske enthaltende Region abgegrenzt wird. Diese Region ist dabei größer als die Projektionsmaske selbst. Es kann sich also beispielsweise um einen rechteckigen Bereich handeln, in welchem sich die Projektionsmaske befindet. Z.B. kann eine sogenannte Bounding-Box verwendet werden. Nun wird iterativ in zumindest einer Iteration die folgende Schrittfolge durchgeführt. Aus Pixelwerten von Pixeln der Region werden insgesamt eine Metall-Zuordnungsfunktion von Pixelwerten zu dem Metallkörper und eine Hintergrund-Zuordnungsfunktion von Pixelwerten zu einem metallfreien Bildhintergrund ermittelt. Jede Zuordnungsfunktion beschreibt also jeweils für mögliche Pixelwerte, also beispielsweise Pixelwerte aus einem Werteintervall, ob der jeweilige Pixelwert repräsentativ oder typisch für den Metallkörper beziehungsweise den metallfreien Bildhintergrund ist. Es kann sich hierbei um eine binäre Zuordnung handeln (0 oder 1) oder auch eine mehrwertige Angabe, beispielsweise eine Wahrscheinlichkeit oder einen Prozentsatz. Zu jedem Pixel der Region wird dann anhand seines jeweiligen Pixelwerts und auf Grundlage der beiden Zuordnungsfunktionen (Metall-Zuordnungsfunktion und Hintergrund-Zuordnungsfunktion) seine Zugehörigkeit zu der Projektionsmaske festgelegt. Die Projektionsmaske beschreibt all diejenigen Pixel, die als zum Metallobjekt gehörig angesehen werden. Durch die Weiterbildung ergibt sich der Vorteil, dass man nun in den artefaktfreien Projektionsbildern jeweils für eine Region auf Grundlage der darin enthaltenen Pixelwerte eine Unterscheidung zwischen Pixelwerten des Metallkörpers und Pixelwerten des metallfreien Bildhintergrunds vornimmt. Durch Vorsehen einer Region, die kleiner als das gesamte Projektionsbild, aber größer als die Projektionsmaske ist, wird verhindert, dass die Vielzahl der metallfreien Pixelwerte statistisch überwiegt und somit die Unterscheidung verfälscht.

Zu der Erfindung gehören auch vorteilhafte Weiterbildungen, durch deren Merkmale sich zusätzliche Vorteile ergeben.

Eine oder beide Zuordnungsfunktionen sind jeweils insbesondere Log-Likelihood-Funktionen. Die Zugehörigkeit jedes Pixels zu der Projektionsmaske wird dann festgelegt, indem aus seinem Pixelwert der jeweilige Log-Likelihood-Wert für Metall und Hintergrund ermittelt wird und diese Log-Likelihood-Werte verglichen werden. Ist der Log-Likelihood-Wert für Metall größer als der für den Bildhintergrund, so wird das Pixel der Projektionsmaske zugeordnet. Andernfalls wird es von der Projektionsmaske ausgeschlossen. Die Verwendung von Log-Likelihood-Funktionen weist den Vorteil auf, dass eine statistisch optimale Unterscheidung zwischen statistisch verteilten, beispielsweise gaußverteilten, Pixelwerten zweier unterschiedlicher Gruppen (Metall und Bildhintergrund) ermittelt werden kann.

Eine Weiterbildung hierzu sieht vor, dass die Metall-Zuordnungsfunktion auf der Grundlage eines statistischen Mittelwerts und/oder einer statistischen Varianz der Pixelwerte der von der Projektionsmaske umfassten Pixel ermittelt wird. Mit anderen Worten wird das durch die Projektionsmaske repräsentierte Wissen über die Pixelwerte des Metallobjekts statistisch durch den Mittelwert $\mu_M$ und/oder die Varianz $\sigma_M$ repräsentiert und beim Zuordnen der Pixel in der aktuellen Iteration genutzt. Zusätzlich oder alternativ dazu kann auch die Hintergrund-Zuordnungsfunktion auf der Grundlage eines statistischen Mittelwerts $\mu_B$ und/oder einer statistischen Varianz $\sigma_B$ der Pixelwerte der von der Projektionsmaske ausgeschlossenen Pixel ermittelt werden. Hierdurch ist dann auch die Statistik der Pixelwerte des Bildhintergrunds, wie er durch die aktuelle Projektionsmaske beschrieben ist, genutzt.

Eine Weiterbildung sieht vor, dass eine oder beide Zuordnungsfunktionen jeweils auf der Grundlage einer Kantendetektion gebildet werden. Hierdurch ergibt sich der Vorteil, dass die in den Projektionsbildern erkennbaren Kanten des Metallobjekts genutzt werden, um die Pixel korrekt als Metall oder Bildhintergrund einzuordnen.

Eine Weiterbildung sieht vor, dass durch eine oder beide Zuordnungsfunktionen jeweils für jede Pixelposition eine Nachbarschaftsbeschreibung berücksichtigt wird. Es wird also beachtet, dass ein einzelnes Pixel allein nicht das Metallobjekt ausmachen kann, sondern auch benachbarte Pixel vorhanden sein sollten oder sind, die dem Metallobjekt zugehören. Die besagte Nachbarschaftsbeschreibung ist entsprechend abhängig von einer Anzahl der angrenzenden und von der Projektionsmaske umfassten Pixel (Pixel des Metallobjekts) einerseits und einer Anzahl der angrenzenden und von der Projektionsmaske ausgeschlossenen Pixel (Bildhintergrund) andererseits. Die Nachbarschaftsbeschreibung ist z.B. als ein Region-Growing für die Projektionsmaske oder für die inverse Projektionsmaske realisiert. Eine besonders einfach zu implementierende und bevorzugte Ausführungsform hierzu sieht aber vor, dass die Nachbarschaftsbeschreibung durch Glätten der aktuellen Projektionsmaske ermittelt wird. Beispielsweise kann hierzu ein zweidimensionales Gauß-Filter oder ein anderes zweidimensionales Tiefpass-Filter verwendet werden, welches auf die Projektionsmaske angewendet werden kann.

Eine Weiterbildung sieht vor, dass eine oder beide Zuordnungsfunktionen jeweils in Abhängigkeit von der ursprünglichen oder nicht-angepassten Projektionsmaske gebildet werden. Mit anderen Worten wird der Verlauf oder die Form der initialen, direkt durch Projizieren der ersten Volumenmaske gebildeten Projektionsmaske berücksichtigt. Hierdurch ergibt sich der Vorteil, dass die anfängliche Information (a-priori-Information) beibehalten wird. Mit anderen Worten wird ein Entarten der Form der Projektionsmaske, also eine von dem Metalobjekt unabhängige Formveränderung, verhindert.

Die beschriebenen Weiterbildungen, die bei den Zuordnungsfunktionen berücksichtigt werden können, können kombiniert werden, beispielsweise als gewichtete Summe.

Wie bereits ausgeführt, werden die angepassten Projektionsmasken wieder zu der zweiten Volumenmaske zusammengefügt. Das Verfeinern oder Anpassen der zweiten Volumenmaske erfolgt dann, in dem Volumenmodell vergleichbare Schritte durchgeführt werden, wie sie im Zusammenhang mit der Anpassung der (zweidimensionalen) Projektionsmasken beschrieben sind. Mit anderen Worten wird gemäß einem zweiten Aspekt der Erfindung in dem Volumenmodell eine die zweite Volumenmaske enthaltende Region abgegrenzt werden. Die Region ist dabei größer als die zweite Volumenmaske und kleiner als das gesamte Volumenmodell. Iterativ wird dann in zumindest einer Iteration jeweils folgendes durchgeführt

Aus Voxelwerten der Voxel der Region werden insgesamt eine volumenbasierte Metall-Zuordnungsfunktion von Voxelwerten zu dem Metallkörper und eine volumenbasierte Hintergrund-Zuordnungsfunktion von Voxelwerten zu einem metallfreien Volumenhintergrund ermittelt. Insbesondere handelt es sich bei den beiden volumenbasierten Zuordnungsfunktionen jeweils um eine Log-Likelihood-Funktion. In diesem Fall kann die Zugehörigkeit jedes Voxels zu der zweiten Volumenmaske (d.h. das Voxel wird als zum Metallobjekt zugehörig angesehen) auf der Grundlage eines Vergleichs der aus seinem Voxelwert ermittelten Log-Likelihood-Werte für Metall und Volumenhintergrund festgelegt werden. Der beschriebene Bildhintergrund und der beschriebene Volumenhintergrund beschreibt jeweils metallfreies Körpergewebe oder beispielsweise Knochen. Allgemein wird also bei dieser Weiterbildung in jeder Iteration zu jedem Voxel der Region anhand seines jeweiligen Voxelwerts auf der Grundlage der beiden Zuordnungsfunktionen (volumenbasierte Metall-Zuordnungsfunktion und eine volumenbasierte Hintergrund-Zuordnungsfunktion) erneut seine Zugehörigkeit zu der zweiten Volumenmaske festgelegt.

In der beschriebenen Weise kann auch die volumenbasierte Metall-Zuordnungsfunktion auf der Grundlage eines statistischen Mittelwerts $\mu_M$ und/oder einer statistischen Varianz $\sigma_M$ der Voxelwerte der von der zweiten Volumenmaske umfassten Voxel ermittelt werden. Zusätzlich oder alternativ dazu kann die volumenbasierte Hintergrund-Zuordnungsfunktion auf der Grundlage eines statistischen Mittelwerts $\mu_B$ und/oder einer statistischen Varianz $\sigma_B$ der Voxelwerte der von der zweiten Volumenmaske ausgeschlossenen Voxel ermittelt werden.

Auch die volumenbasierten Zuordnungsfunktionen können jeweils auf der Grundlage einer Kantendetektion gebildet sein. Mit Kanten sind hierbei Oberflächen gemeint, die durch einen vorbestimmten Mindestkontrast der angrenzenden Voxelwerte gekennzeichnet sind.

Eine oder beide volumenbasierte Zuordnungsfunktionen können jeweils auch für jede Voxelposition eine Nachbarschaftsbeschreibung berücksichtigen, welche von einer Anzahl der angrenzenden und von der zweiten Volumenmaske erfassten Voxel (Metallvoxel) einerseits und von einer Anzahl der angrenzenden und von der zweiten Volumenmaske ausgeschlossenen Voxel (Hintergrundvoxel) andererseits abhängig ist. Auch hier kann die Nachbarschaftsbeschreibung in einem besonders einfach zu implementierenden Fall durch Glätten der aktuellen Volumenmaske ermittelt werden. Hierbei sind dann dreidimensionale Tiefpass-Filter zu verwenden, also beispielsweise eine dreidimensionale Gauß-Funktion. Das Glätten kann im zweidimensionalen und im dreidimensionalen Fall jeweils durch Falten (Englisch: Convolution) in an sich bekannter Weise durchgeführt werden.

Um eine Entartung der zweiten Volumenmaske während der Iterationen zu vermeiden, wird gemäß einer Weiterbildung eine oder beide volumenbasierten Zuordnungsfunktionen jeweils in Abhängigkeit von der nicht-angepassten zweiten Volumenmaske gebildet. Es wird also die initiale, ursprüngliche zweite Volumenmaske berücksichtigt.

Die beschriebenen Aspekte der volumenbasierten Zuordnungsfunktionen können alle auch beispielsweise als gewichtete Summe kombiniert sein.

Wie bereits ausgeführt, gehört zu der Erfindung auch die Vorrichtung zum Durchführen des Verfahrens. Erfindungsgemäß ist also eine Vorrichtung zum Abgrenzen eines Metallobjekts in einem aus Projektionsbildern eines das Metallobjekt enthaltenen Körpers gebildeten Volumenmodells bereitgestellt. Die Vorrichtung weist eine Empfangseinrichtung zum Empfangen der Projektionsbilder und eine Prozessoreinrichtung auf. Die Empfangseinrichtung kann beispielsweise eine Leseeinrichtung zum Lesen eines Speichermediums umfassen, auf welchem die Projektionsbilder gespeichert sind. Die Empfangseinrichtung kann auch beispielsweise eine Anschlusseinrichtung zum Anschließen der beschriebenen Projektionseinrichtung, also beispielsweise zum Anschließen eines Computertomographen, sein. Die Prozessoreinrichtung umfasst einen Programmcode, der dazu eingerichtet ist, bei Ausführen durch die Prozessoreinrichtung eine Ausführungsform des erfindungsgemäßen Verfahrens durchzuführen.

Im Folgenden ist ein Ausführungsbeispiel der Erfindung beschrieben. Hierzu zeigt:

FIG 1    eine schematische Darstellung einer Ausführungsform der erfindungsgemäßen Vorrichtung;

FIG 2    ein Flussschaudiagramm zum Veranschaulichen einer Ausführungsform des erfindungsgemäßen Verfahrens;

FIG 3    eine Skizze zur Veranschaulichung eines Verfahrensschritts einer Ausführungsform des erfindungsgemäßen Verfahrens, durch welchen eine Projektionsmaske angepasst wird;

FIG 4    eine Skizze zur Veranschaulichung eines Verfahrensschritts des Verfahrens, durch welchen iterativ eine Volumenmaske angepasst wird.

Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

In den Figuren sind funktionsgleiche Elemente jeweils mit denselben Bezugszeichen versehen.

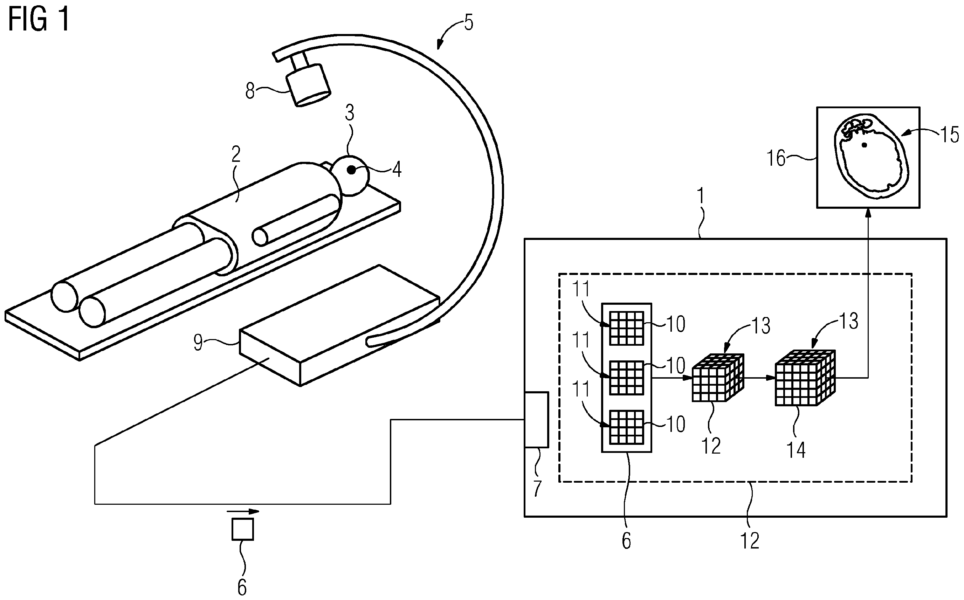

FIG 1 zeigt eine Vorrichtung 1, mittels welcher ein Körper 2 eines Patienten untersucht werden kann. Beispielsweise kann ein Kopf 3 untersucht werden. Ein Problem hierbei kann sein, dass beispielsweise in dem Kopf 3 oder allgemein in dem Körper 2 ein Metallobjekt 4 enthalten sein kann, welches das Abbilden des Körpers 2 stört.

Zum Abbilden des Körpers 2, beispielsweise des Kopfes 3, kann die Vorrichtung 1 mit einer Projektionseinrichtung 5 gekoppelt sein, bei der es sich beispielsweise um einen Computertomographen, insbesondere eine Röntgen-C-Bogen-Anlage, handeln kann. Aus der Projektionseinrichtung 5 kann die Vorrichtung 1 Bilddaten 6 empfangen. Hierzu kann eine entsprechende Empfangseinrichtung 7 in der Vorrichtung 1 bereitgestellt sein. Die Empfangseinrichtung 7 kann die Bilddaten 6 beispielsweise direkt über ein Kabel oder eine Funkverbindung aus der Projektionseinrichtung 5 empfangen oder aus einem Speichermedium auslesen, welches die Bilddaten 6 speichert.

Zum Erzeugen der Bilddaten 6 kann die Projektionseinrichtung 5 den Körper 2, beispielsweise den Kopf 3, mittels einer Röntgenquelle 8 mit Röntgenstrahlen bestrahlen, so dass der Körper 2 mit dem darin enthaltenen Metallobjekt 4 auf einem Röntgen-Detektor 9 als Projektion abgebildet wird.

Die Bilddaten 6 umfassen somit Projektionsbilder 10 aus unterschiedlichen Bestrahlungswinkeln oder Projektionswinkeln. Jedes der Projektionsbilder 10 enthält wiederum einzelne Pixel 11, deren Pixelwerte jeweils eine durch den Körper 2 bewirkte Dämpfung der Strahlung der Projektionseinrichtung 5 beschreiben.

Die Vorrichtung 1 kann eine Prozessoreinrichtung 12 aufweisen, durch welche aus den Projektionsbildern 10 ein erstes Volumenmodell 12 in an sich bekannter Weise beispielsweise auf der Grundlage der Methode der gefilterten Rückprojektion berechnet werden kann. Das Volumenmodell 12 beschreibt den in den Projektionsbildern 10 abgebildeten Teil des Körpers 2 dreidimensional. Das Volumenmodell 12 enthält hierzu Voxel 13, deren Voxelwerte zu einzelnen Volumenbereichen jeweils die Dämpfungseigenschaft des Körpers 2 bezüglich der Strahlung der Projektionseinrichtung 5 beschreibt.

Die Prozessoreinrichtung 12 führt auf der Grundlage des Volumenmodells 12 ein im Folgenden beschriebenes Verfahren durch, welches es ermöglicht, ein finales Volumenmodell 14 zu berechnen, dessen Voxel 13 Voxelwerte aufweisen, die bezüglich der Voxel 13 des ersten Volumenmodells 12 einen geringeren Artefaktanteil von Metallartefakten des Metallobjekts 4 aufweisen. Auf Grundlage des finalen Volumenmodells 14 können dann Tomographiebilder 15 ermittelt und beispielsweise auf einer Anzeigeeinrichtung 16 angezeigt werden.

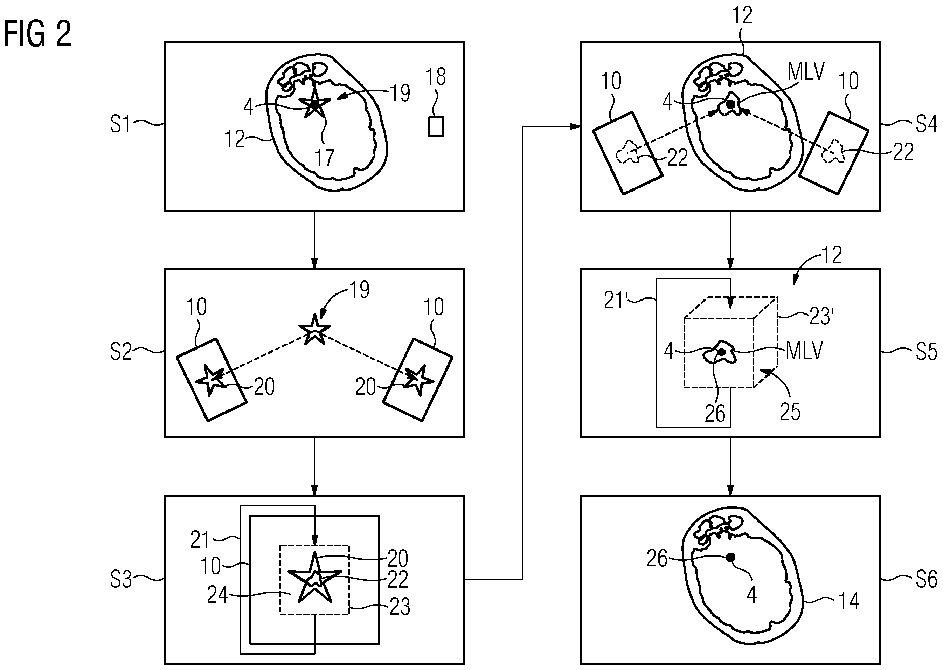

FIG 2 veranschaulicht das durch die Prozessoreinrichtung 12 durchgeführte Verfahren zum Ermitteln des finalen Volumenmodells 14. In einem Schritt S1 wird in der beschriebenen Weise aus den Projektionsbildern 10 das erste Volumenmodell 12 berechnet, das in FIG 2 durch ein einzelnes Tomographiebild 15 repräsentiert ist, wie es aus dem Volumenmodell 12 berechnet werden könnte. Durch das Metallobjekt 4 sind um dieses herum die Voxelwerte der angrenzenden Voxel 13 verfälscht, so dass sich die dargestellten Metallartefakte 17 ergeben. Alle zu dem Metallobjekt 4 und den Metallartefakten 17 gehörenden Voxel 13 können auf der Grundlage eines Segmentierungskriteriums 18 erkannt oder segmentiert werden, das heißt von den übrigen Voxeln 13 des Volumenmodells 12 unterschieden werden. Beispielsweise kann eine Schwellwertdetektion durchgeführt werden. Die hierdurch selektierten oder detektierten Voxel 13 bilden eine erste Volumenmaske 19.

In einem Schritt S2 kann eine initiale Projektion dieser ersten Volumenmaske 19 beispielsweise auf der Grundlage der bekannten Vorwärtsprojektion auf die Projektionsbilder 10 erfolgen. Mit anderen Worten wird in den Projektionsbildern 10 die jeweilige Projektion der Volumenmaske 19 ermittelt oder abgegrenzt, d.h. es werden diejenigen Pixel in den Projektionsbildern 10 identifiziert, die das Abbild oder die Projektion der Volumenmaske 19 darstellen. Hierdurch erhält man in jedem Projektionsbild 10 eine jeweilige Projektionsmaske 20.

In einem Schritt S3 wird in jedem Projektionsbild 10, das heißt auf Grundlage der jeweiligen Pixel oder Bilddaten des jeweiligen Projektionsbilds 10, in mindestens einer Iteration 21 aus der jeweiligen Projektionsmaske 20 eine angepasste Projektionsmaske 22 ermittelt. Hierzu wird eine Region 23, welche die Projektionsmaske 20 und einen Teil eines Bildhintergrunds 24 enthält, ermittelt. Die Region 23 ist größer, umfasst also mehr Pixel, als die Projektionsmaske 20 selbst.

Die jeweiligen angepassten Projektionsmasken 22 aller Projektionsbilder 10 können in einem Schritt S4 beispielsweise auf der Grundlage der bekannten Rückprojektion in dem Volumenmodell 12 zu einer zweiten Volumenmaske MLV kombiniert werden, welche eine dreidimensionale angepasste Formbeschreibung des Metallobjekts 4 darstellt.

In einem Schritt S5 wird die zweite Volumenmaske MLV in mindestens einer Iteration 21' auf der Grundlage der Voxelwerte der Voxel 13 einer Region 23' des Volumens 12 angepasst. Die Region 23' ist größer als die zweite Volumenmaske MLV, enthält also mehr Voxel als diese. Hierdurch ergibt sich zusätzlich zu der Volumenmaske MLV ein metallfreier Volumenhintergrund 25. Es ergibt sich eine angepasste Volumenmaske 26 als Ergebnis der zumindest einen Iteration 21'. Die angepasste Volumenmaske 26 beschreibt die Form des Metallobjekts 4 genauer als die initiale Volumenmaske 19.

In einem Schritt S6 kann auf Grundlage der Formbeschreibung des Metallobjekts 4, das heißt auf Grundlage der angepassten zweiten Volumenmaske 26 in an sich bekannter Weise das finale Volumenmodell 14 berechnet werden.

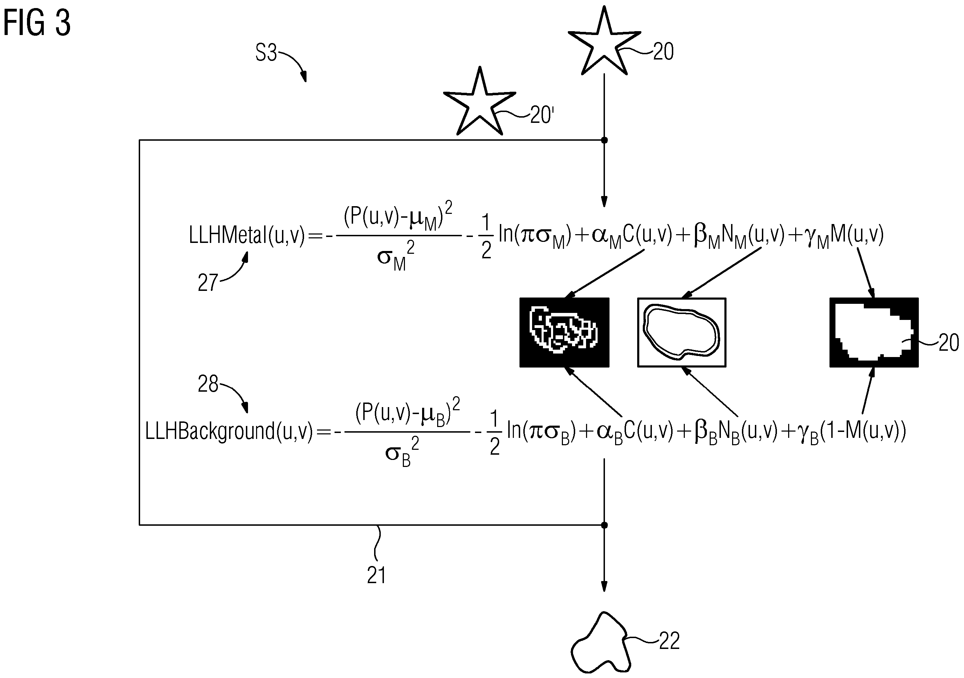

FIG 3 veranschaulicht zum Schritt S3 wie aus der Projektionsmaske 20 mittels der zumindest einen Iterationen 21 die angepasste Projektionsmaske 22 ermittelt werden kann.

Es kann zu Pixelwerten P(u, v), die sich innerhalb der in der aktuellen Iteration 21 zugrundegelegten aktuelle Projektionsmaske 20' befinden, ein statistischer Mittelwert $\mu_M$ (M-Metall) und eine Varianz $\sigma_M$ ermittelt werden. Die Koordinaten u, v geben jeweils die Position des Pixels mit dem Pixelwert P in dem Projektionsbild 10 an. Zusätzlich kann eine Kanteninformation C(u, v) berücksichtigt werden. Die Kantendetektion kann beispielsweise auf der Grundlage des Algorithmus Canny-Kanten-Detektion ermittelt werden. Es kann auch eine Nachbarschaftsbeschreibung $N_M(u, v)$

berücksichtigt werden. Zusätzlich kann auch die initiale Projektionsmaske 20, M(u,v), zugrundegelegt werden, wobei M(u, v) die binäre Angabe (0/1) betreffend die Zugehörigkeit zur initialen Projektionsmaske 20 darstellt. Die beschriebenen Aspekte können durch Gewichtungsfaktoren $\alpha_M$, $\beta_M$, $\gamma_M$ in der in FIG 3 veranschaulichten Weise kombiniert werden. Insgesamt ergibt sich hierdurch eine Metall-Zuordnungsfunktion 27, LLHMetal(u, v), die eine Log-Likelihood-Funktion darstellt.

Diese Metall-Zuordnungsfunktion 27 kann ergänzt werden durch eine Hintergrund-Zuordnungsfunktion 28, LLHBackground(u,v), die in den gezeigten Fall ebenfalls eine Log-Likelihood-Funktionen darstellt. Die Bildhintergrund-Zuordnungsfunktion 28 ergibt zu jedem Pixel einen Log-Likelihood-Wert LLHBack-ground(u, v), der auf Grundlage der folgenden Aspekte gebildet werden kann. Es können ein Mittelwert $\mu_B$ und eine Varianz $\sigma_B$ aus den Pixelwerten P(u, v) derjenigen Pixel ermittelt werden, die sich außerhalb der aktuellen Projektionsmaske 20' befinden. Des Weiteren können wieder die Kantendetektion mit der Kanteninformation C(u, v) und/oder eine inverse Nachbarschaftsbeschreibung $N_B$ und/oder die Inverse der initialen Projektionsmaske 20, berechnet durch 1-M(u, v), verwendet werden.

Zum Bilden der Projektionsmaske 20' der Folgeiteration wird zu jedem Pixel auf Grundlage seines Pixelwerts P(u, v) der jeweilige Funktionswert der beiden Zuordnungsfunktionen 27, 28 ermittelt und die Projektionsmaske 20' aus denjenigen Pixeln ermittelt, für die gilt:

$$LLHMetal(u, v) > LLHBackground(u, v).$$

Die Projektionsmasken 20, 20', 22 können binäre Masken (Maskenwerte der Masken: 0 oder 1) sein.

Die Zahl der Iterationen 21 kann vorgegeben sein und beispielsweise in einem Bereich von 1 bis 20 liegen. Es kann auch vorgesehen sein, ein Abbruchkriterium zugrundezulegen, beispielsweise die minimale Veränderung der Projektionsmaske 20' beispielsweise in einer Anzahl Pixel. Nach Ende der letzten Iteration 21 ergibt sich die angepasste Projektionsmaske 22.

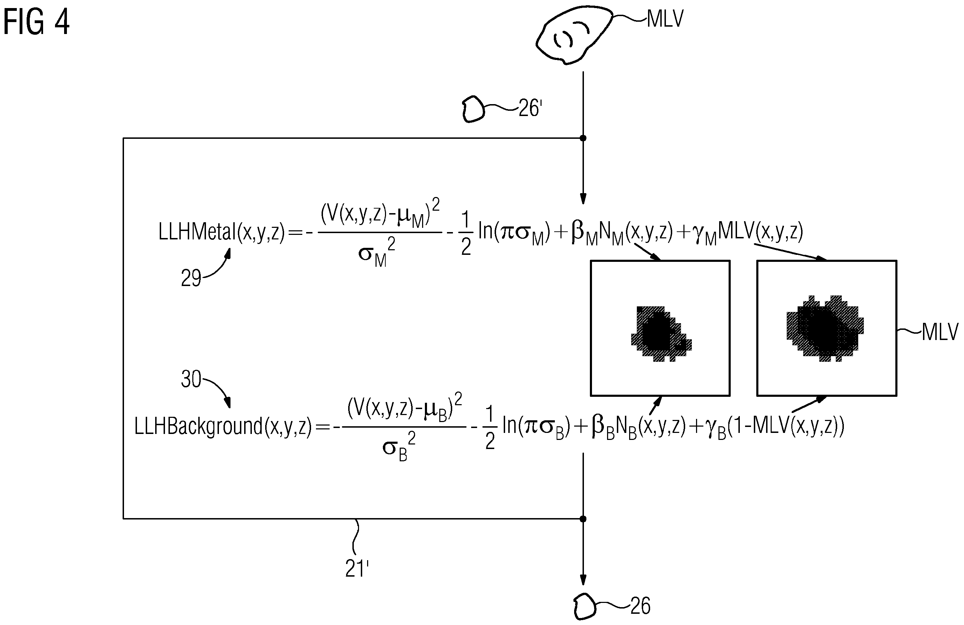

FIG 4 veranschaulicht analog zu FIG 3 die Anpassung der zweiten Volumenmaske MLV, um hieraus die angepasste Volumenmaske 26 zu ermitteln. Jedes Voxel der Region 23' weist einen Voxelwert V(x, y, z) auf, wobei x, y, z die Koordinaten des Voxels in dem Volumenmodell 12 darstellen. Zu jedem Voxel können eine volumenbasierte Metall-Zuordnungsfunktion 29, LLHMe-tal(x, y, z), und eine volumenbasierte Hintergrund-Zuordnungsfunktion 30, LLHBackground(x, y, z), ermittelt werden. Es handelt sich auch hierbei um Log-Likelihood-Funktionen. Auf Grundlage der aktuellen Volumenmaske 26' können ein Mittelwert $\mu_M$ und eine Varianz $\sigma_M$ der Voxelwerte der Voxel der Volumenmaske 26' ermittelt werden. Es können wieder die beschriebene Nachbarschaftsbeschreibung $N_M(x, y, z)$ und die initiale Volumenmaske MLV zugrundegelegt werden. In FIG 4 nicht dargestellt, aber dennoch berücksichtigbar ist die Kanteninformation C(x, y, z). Es können wieder Gewichtungfaktoren $\beta_M$ und $\gamma_M$ zum gewichteten Kombinieren der Askpekte vorgesehen sein.

Entsprechend können für Voxel außerhalb der aktuellen Volumenmaske 26' ein Mittelwert $\mu_B$ und eine Varianz $\sigma_B$ zugrundegelegt werden. Es können auch wieder die inverse Nachbarschaftsinformation $N_B(x, y, z)$ und die inverse initiale Volumenmaske 1-MLV (x, y, z) zugrundegelegt werden und wieder eine Gewichtung $\beta_B$ und $\gamma_B$ zum Kombinieren der Aspekte verwendet werden.

Die Zuordnung eines Voxels zur Volumenmaske 26' und in der letzten Iteration 21' zur Volumenmaske 26 kann wieder durch Vergleich erfolgen:

$$LLHMetal(x, y, z) > LLHBackground(x, y, z).$$

Auch die Volumenmasken MLV, 26' und 26 können binäre Masken sein (Voxelwerte der Masken: 0 oder 1).

Das Verfahren lässt sich mit geringem Berechnungsaufwand durchführen, da die betrachtete Regionen 23, 23' im Verhältnis zu den Projektionsbildern 10 und dem Volumenmodell 12 klein sind, beispielsweise weniger als 20 Prozent, insbesondere weniger als 10 Prozent, der Pixel beziehungsweise Voxel enthalten.

Insgesamt zeigt das Beispiel, wie durch die Erfindung eine automatisierte Reduktion von Metallartefakten durch eine kombinierte Segmentierung in der 3D-Volumendomäne und der Projektionsdomäne bereitgestellt werden kann.

**Patentansprüche**

1. Verfahren zum Abgrenzen eines Metallobjekts (4) für eine Artefaktreduktion in Tomographiebildern (15), wobei

- Projektionsbilder (10) eines das Metallobjekt (4) enthaltenden Körpers (2) aus einer Projektionseinrichtung (5) empfangen werden, und

- auf der Grundlage der Projektionsbilder (10) ein Volumenmodell (12) zumindest eines Teils des Körpers (2) gebildet wird,
- in dem Volumenmodell (12) auf der Grundlage eines Segmentierungskriteriums (18) Voxel (13), die Teil eines das Metallobjekt (4) und zumindest ein Metallartefakt (17) umfassenden Bereichs sind, ausgewählt und zu einer ersten Volumenmaske (19) kombiniert werden,
- die erste Volumenmaske (19) auf die Projektionsbilder (10) projiziert und hierdurch eine jeweilige Projektionsmaske (20) erzeugt wird,
- jede Projektionsmaske (20) auf der Grundlage von Pixelwerten der jeweiligen Projektionsbilder (10) angepasst wird,
- aus den angepassten Projektionsmasken (22) eine zweite Volumenmaske (MLV) ermittelt wird,
- die zweite Volumenmaske (MLV) auf der Grundlage von Voxelwerten des Volumenmodells (12) angepasst wird,
- die angepasste Volumenmaske (12) als Beschreibung einer dreidimensionalen Form des Metallobjekts (4) bereitgestellt wird,

wobei das Anpassen der Projektionsmasken (20) jeweils erfolgt, indem in dem jeweiligen Projektionsbild (10) eine die Projektionsmaske (20) enthaltende Region (23) abgegrenzt wird, die kleiner als das gesamte Projektionsbild (10), aber größer als die Projektionsmaske (20) ist, und iterativ in zumindest einer Iteration (21) jeweils: aus Pixelwerten von Pixeln (11) der Region (23) insgesamt eine Metall-Zuordnungsfunktion (27) von Pixelwerten zu dem Metallkörper (4) und eine Hintergrund-Zuordnungsfunktion (28) von Pixelwerten zu einem metallfreien Bildhintergrund (24) ermittelt wird und zu jedem Pixel (11) der Region (23) anhand seines jeweiligen Pixelwerts und auf der Grundlage der beiden Zuordnungsfunktionen (27, 28) erneut seine Zugehörigkeit zu der Projektionsmaske (20, 20', 22) festgelegt wird, und wobei das Anpassen der zweiten Volumenmaske (MLV) jeweils erfolgt, indem in dem Volumenmodell (12) eine die zweite Volumenmaske (MLV) enthaltende Region (23'), die größer als die zweite Volumenmaske (MLV) und kleiner als das gesamte Volumenmodell (12) ist, abgegrenzt wird, und iterativ in zumindest einer Iteration jeweils: aus Voxelwerten von Voxeln (13) der Region (23') insgesamt eine volumenbasierte Metall-Zuordnungsfunktion (29) von Voxelwerten zu dem Metallkörper (4) und eine volumenbasierte Hintergrund-Zuordnungsfunktion (30) von Voxelwerten zu einem metallfreien Volumenhintergrund (25) ermittelt wird und zu jedem Voxel (13) der Region (23') anhand seines jeweiligen Voxelwerts und auf der Grundlage der beiden Zuordnungsfunktionen (29, 30) erneut seine Zugehörigkeit zu der zweiten Volumenmaske (26', 26) festgelegt wird.

2. Verfahren nach Anspruch 1, wobei als das Segmentierungskriterium (18) eine Schwellwertdetektion verwendet wird.

3. Verfahren nach einem der vorhergehenden Ansprüche, wobei als eine oder beide Zuordnungsfunktionen (27, 28) jeweils eine Log-Likelihood-Funktion ermittelt wird und die Zugehörigkeit jedes Pixels (11) zu der Projektionsmaske (20, 20', 22) auf der Grundlage eines Vergleichs der aus seinem Pixelwert ermittelten Log-Likelihood-Werte für Metall und Bildhintergrund festgelegt wird.

4. Verfahren nach einem der vorhergehenden Ansprüche, wobei die Metall-Zuordnungsfunktion (27) auf der Grundlage eines statistischen Mittelwerts und/oder einer statistische Varianz der Pixelwerte der von der Projektionsmaske (20, 20') umfassten Pixel (11) ermittelt wird und/oder die Hintergrund-Zuordnungsfunktion (28) auf der Grundlage eines statistischen Mittelwerts und/oder einer statistische Varianz der Pixelwerte der von der Projektionsmaske (20, 20') ausgeschlossenen Pixel (11) ermittelt wird.

5. Verfahren nach einem der vorhergehenden Ansprüche, wobei eine oder beide Zuordnungsfunktionen (27, 28) jeweils auf der Grundlage einer Kantendetektion (C) gebildet werden.

6. Verfahren nach einem der vorhergehenden Ansprüche, wobei eine oder beide Zuordnungsfunktionen (27, 28) jeweils für jede Pixelposition eine Nachbarschaftsbeschreibung (N) berücksichtigen, welche von einer Anzahl der angrenzenden, von der Projektionsmaske (20, 20') umfassten Pixel (11) einerseits und einer Anzahl der angrenzenden, von der Projektionsmaske (20, 20') ausgeschlossenen Pixel (11) andererseits abhängig ist.

7. Verfahren nach einem der vorhergehenden Ansprüche, wobei eine oder beide Zuordnungsfunktionen (27, 28) jeweils in Abhängigkeit von der nicht-angepassten Projektionsmaske (20) gebildet werden.

8. Verfahren nach einem der vorhergehenden Ansprüche, wobei als eine oder beide volumenbasierten Zuordnungsfunktionen (29, 30) jeweils eine Log-Likelihood-Funktion ermittelt wird und die Zugehörigkeit jedes Voxels (13) zu der zweiten Volumenmaske (26, 26') auf der Grundlage eines Vergleichs der aus seinem Voxelwert ermittelten Log-Likelihood-Werte für Metall und Volumenhintergrund festgelegt wird.

9. Verfahren nach einem der vorhergehenden Ansprüche, wobei die volumenbasierte Metall-Zuordnungsfunktion (29) auf der Grundlage eines statistischen Mittelwerts und/oder einer statistische Varianz der Voxelwerte der von der zweiten Volumenmaske (MLV, 26') umfassten Voxel (13) ermittelt wird und/oder die volumenbasierte Hintergrund-Zuordnungsfunktion (30) auf der Grundlage eines statistischen Mittelwerts und/oder einer statistische Varianz der Voxelwerte der von der zweiten Volumenmaske (MLV, 26') ausgeschlossenen Voxel (13) ermittelt wird.

10. Verfahren nach einem der vorhergehenden Ansprüche, wobei eine oder beide volumenbasierten Zuordnungsfunktionen (29, 30) jeweils auf der Grundlage einer Kantendetektion (C) gebildet werden.

11. Verfahren nach einem der vorhergehenden Ansprüche, wobei eine oder beide volumenbasierte Zuordnungsfunktionen (29, 30) jeweils für jede Voxelposition eine Nachbarschaftsbeschreibung (N) berücksichtigen, welche von einer Anzahl der angrenzenden, von der zweiten Volumenmaske (MLV, 26') umfassten Voxel (13) einerseits und einer Anzahl der angrenzenden, von der zweiten Volumenmaske (MLV, 26') ausgeschlossenen Voxel (13) andererseits abhängig ist.

12. Verfahren nach einem der vorhergehenden Ansprüche, wobei eine oder beide volumenbasierten Zuordnungsfunktionen (29, 30) jeweils in Abhängigkeit von der nicht-angepassten zweiten Volumenmaske (MLV) gebildet werden.

13. Vorrichtung zum Abgrenzen eines Metallobjekts (4) in einem aus Projektionsbildern (10) eines das Metallobjekt (4) enthaltenden Körpers gebildeten Volumenmodells (12), aufweisend eine Empfangseinrichtung (7) zum Empfangen der Projektionsbilder (10) und eine Prozessoreinrichtung (12), die Programmcode aufweist, der dazu eingerichtet ist, bei Ausführen durch die Prozessoreinrichtung (12) ein Verfahren nach einem der vorhergehenden Ansprüche durchzuführen.

## Claims

1. Method for delineating a metal object (4) for artifact reduction in tomography images (15), wherein

   - projection images (10) of a body (2) containing the metal object (4) are received from a projection device (5), and
   - a volume model (12) of at least part of the body (2) is formed on the basis of the projection images (10),
   - voxels (13), which are part of a region encompassing the metal object (4) and at least one metal artifact (17), are chosen in the volume model (12) on the basis of a segmentation criterion (18) and combined to form a first volume mask (19),
   - the first volume mask (19) is projected onto the projection images (10) and a respective projection mask (20) is generated hereby,
   - each projection mask (20) is adjusted on the basis of pixel values of the respective projection images (10),
   - a second volume mask (MLV) is determined from the adjusted projection masks (22),
   - the second volume mask (MLV) is adjusted on the basis of voxel values of the volume model (12),
   - the adjusted volume mask (12) is supplied as a description of a three-dimensional form of the metal object (4),

   wherein the projection masks (20) are each adjusted in that a region (23) containing the projection mask (20) is delineated in the respective projection image (10), which region (23) is smaller than the overall projection image (10), but larger than the projection mask (20), and iteratively in at least one iteration (21) in each case:
   overall, a metal allocation function (27) of pixel values to the metal body (4) and a background allocation function (28) of pixel values to a metal-free image background (24) is determined from pixel values of pixels (11) of the region (23) and for each pixel (11) of the region (23) the affiliation thereof to the projection mask (20, 20', 22) is defined again using its respective pixel value and on the basis of the two allocation functions (27, 28) and
   wherein the second volume mask (MLV) is in each case adjusted in that in the volume model (12) a region (23') containing the second volume mask (MLV) is delineated, which region (23') is larger than the second volume mask (MLV) and smaller than the overall volume model (12), and iteratively in at least one iteration in each case:
   overall, a volume-based metal allocation function (29) of voxel values to the metal body (4) and a volume-based background allocation function (30) of voxel values to a metal-free volume background (25) is determined from voxel values of voxels (13) of the region (23') and for each voxel (13) of the region (23') the affiliation thereof to the second volume mask (26', 26) is defined again using its respective voxel value and on the basis of the two allocation functions (29, 30).

2.  Method according to claim 1, wherein a threshold value detection is used as the segmentation criterion (18).

3.  Method according to one of the preceding claims, wherein a log-likelihood function is in each case determined as one or both allocation function(s) (27, 28) and the affiliation of each pixel (11) with the projection mask (20, 20', 22) is defined on the basis of a comparison of the log-likelihood values for metal and image background determined from its pixel value.

4.  Method according to one of the preceding claims, wherein the metal allocation function (27) is determined on the basis of a statistical mean and/or a statistical variance of the pixel values of the pixels (11) encompassed by the projection mask (20, 20') and/or the background allocation function (28) is determined on the basis of a statistical mean and/or a statistical variance of the pixel values of the pixels (11) excluded from the projection mask (20, 20').

5.  Method according to one of the preceding claims, wherein one or both allocation function(s) (27, 28) is/are each formed on the basis of an edge detection (C).

6.  Method according to one of the preceding claims, wherein one or both allocation function(s) (27, 28) consider(s) a vicinity description (N) for each pixel position, which is dependent on a number of adjoining pixels (11) encompassed by the projection mask (20, 20') on the one hand and a number of adjoining pixels (11) excluded from the projection mask (20, 20') on the other hand.

7.  Method according to one of the preceding claims, wherein one or both allocation function(s) (27, 28) is/are each formed as a function of the non-adjusted projection mask (20).

8.  Method according to one of the preceding claims, wherein a log-likelihood function is in each case determined as one or both volume-based allocation function(s) (29, 30) and the affiliation of each voxel (13) with the second volume mask (26, 26') is defined on the basis of a comparison of the log-likelihood values for metal and volume background determined from its voxel value.

9.  Method according to one of the preceding claims, wherein the volume-based metal allocation function (29) is determined on the basis of a statistical mean and/or a statistical variance of the voxel values of the voxels (13) encompassed by the second volume mask (MLV, 26') and/or the volume-based background allocation function (30) is determined on the basis of a statistical mean and/or a statistical variance of the voxel values of the voxels (13) excluded from the second volume mask (MLV, 26').

10. Method according to one of the preceding claims, wherein one or both volume-based allocation functions (29, 30) is/are each formed on the basis of an edge detection (C).

11. Method according to one of the preceding claims, wherein one or both volume-based allocation function(s) (29, 30) consider(s) a vicinity description (N) for each voxel position, which description is dependent on a number of adjoining voxels (13) encompassed by the second volume mask (MLV, 26') on the one hand and a number of adjoining voxels (13) excluded from the second volume mask (MLV, 26') on the other hand.

12. Method according to one of the preceding claims, wherein one or both volume-based allocation functions (29, 30) are each formed as a function of the non-adjusted second volume mask (MLV).

13. Device for delineating a metal object (4) in a volume model (12) formed from projection images (10) of a body containing the metal object (4), having a receiving device (7) for receiving the projection images (10) and a processor device (12), having program code which is adapted to carry out a method according to one of the preceding claims when run by the processor device (12).

**Revendications**

1.  Procédé de délimitation d'un objet (4) métallique, afin de réduire des artefacts dans des images (15) tomographiques, dans lequel

- on reçoit, d'un dispositif (5) de projection, des images (10) de projection d'un corps (2) contenant l'objet (4) métallique et
- sur la base des images (10) de projection, on forme un modèle (12) en volume d'au moins une partie du corps (2),
- dans le modèle (12) en volume, on choisit, sur la base d'un critère (18) de segmentation, des voxels (13), qui font partie d'une région comprenant l'objet (4) métallique et au moins un artefact (17) métallique et on les combine en un premier masque (19) en volume,
- on projette le premier masque (19) en volume sur les images (10) de projection et on produit ainsi un masque (20) respectif de projection,
- on adapte chaque masque (20) de projection sur la base de valeurs de pixel des images (10) de projection respectives,
- à partir des masques (22) de projection adaptés, on détermine un deuxième masque (MLV) en volume,
- on adapte le deuxième masque (MLV) en volume sur la base de valeurs de voxel du modèle (12) en volume,
- on met le masque (12) en volume adapté à disposition, comme description d'une forme tridimensionnelle de l'objet (4) métallique,

dans lequel l'adaptation des masques (20) de projection s'effectue, respectivement, en délimitant, dans l'image (10) de projection respective, une région (23) contenant le masque (20) de projection, qui est plus petite que l'ensemble de l'image (10) de projection, mais plus grande que le masque (20) de projection et, itérativement, en suivant au moins une itération (21) :
à partir de valeurs de pixel (11) de la région (23), on détermine une fonction (27) d'association au métal de valeurs de pixel par rapport au corps (4) métallique et une fonction (28) d'association à l'arrière-plan de valeurs de pixel par rapport à un arrière-plan (24) d'image sans métal et on fixe, pour chaque pixel (11) de la région (23), à l'aide de sa valeur de pixel et sur la base des deux fonctions (27, 28) d'association, à nouveau son appartenance au masque (20, 20', 22) de projection, et
dans lequel l'adaptation du deuxième masque ( MLV ) en volume s'effectue, respectivement, en délimitant, dans le modèle (12) en volume, une région (23') contenant le deuxième masque (MLV) en volume, qui est plus grande que le deuxième masque (MLV) en volume et plus petite que tout le modèle (12) en volume et, itérativement, en au moins une itération respectivement :
à partir de valeurs de voxel (13) de la région (23') dans son ensemble, on détermine une fonction (29) d'association au métal, reposant sur le volume, de valeurs de voxel par rapport au corps (4) métallique et une fonction (30) d'association à l'arrière-plan, reposant sur le volume, de valeurs de voxel par rapport à un arrière-plan (25) en volume sans métal et pour chaque voxel (13) de la région (23'), on fixe, à l'aide de sa valeur de voxel et sur la base des deux fonctions (29, 30) d'association, à nouveau son appartenance au deuxième masque (26', 26) en volume.

2. Procédé suivant la revendication 1, dans lequel on utilise une détection de valeur de seuil comme critère (18) de segmentation.

3. Procédé suivant l'une des revendications précédentes, dans lequel on détermine, comme l'une ou comme les deux fonctions (27, 28) d'association, respectivement, une fonction de probabilité logarithmique et on fixe l'appartenance de chaque pixel (11) au masque (20, 20', 22) de projection sur la base d'une comparaison des valeurs de probabilité logarithmique déterminées à partir de sa valeur de pixel, pour du métal et pour un arrière-plan d'image.

4. Procédé suivant l'une des revendications précédentes, dans lequel on détermine la fonction (27) d'association au métal sur la base d'une moyenne statistique et/ou d'une variance statistique des valeurs des pixels (11) compris par le masque (20, 20') de projection et/ou on détermine la fonction (28) d'association à l'arrière-plan sur la base d'une moyenne statistique et/ou d'une variance statistique des valeurs des pixels (11) exclus du masque (20, 20') de projection.

5. Procédé suivant l'une des revendications précédentes, dans lequel on forme une ou les deux fonctions (27, 28) d'association, respectivement, sur la base d'une détection (C) de bord.

6. Procédé suivant l'une des revendications précédentes, dans lequel l'une ou les deux fonctions (27, 28) d'association prennent en compte, respectivement, pour chaque position de pixel, une description (N) de voisinage, qui dépend d'un nombre des pixels (11) voisins compris par le masque (20, 20') de projection, d'une part, et d'un nombre des pixels (11) voisins exclus par le masque (20, 20') de projection d'autre part.

7. Procédé suivant l'une des revendications précédentes, dans lequel on forme l'une ou les deux fonctions (27, 28) d'association, respectivement, en fonction du masque (20) de projection qui n'est pas adapté.

**8.** Procédé suivant l'une des revendications précédentes, dans lequel on détermine, comme l'une ou comme les deux fonctions (29, 30) d'association, reposant sur le volume, respectivement, une fonction de probabilité logarithmique et on fixe l'appartenance de chaque voxel (13) au deuxième masque (26, 26') en volume sur la base d'une comparaison entre les valeurs de probabilité logarithmique déterminées à partir de sa valeur de voxel pour du métal et un arrière plan en volume.

**9.** Procédé suivant l'une des revendications précédentes, dans lequel on détermine la fonction (29) d'association au métal, reposant sur le volume, sur la base d'une moyenne statistique et/ou d'une variance statistique des valeurs des voxels (13) compris par le deuxième masque (MLV, 26') en volume et/ou on détermine la fonction (30) d'association à l'arrière-plan, reposant sur le volume, sur la base d'une moyenne statistique et/ou d'une variance statistique des valeurs de voxel (13) exclus du deuxième masque (MLV, 26') en volume.

**10.** Procédé suivant l'une des revendications précédentes, dans lequel on forme l'une ou les deux fonctions (29, 30) d'association, reposant sur le volume, respectivement, sur la base d'une détection (C) de bord.

**11.** Procédé suivant l'une des revendications précédentes, dans lequel une ou les deux fonctions (29, 30) d'association, reposant sur le volume, tiennent compte, pour chaque position de voxel, d'une description de proximité (N), qui dépend d'un nombre des voxels (13) voisins compris par le deuxième masque (MLV, 26') en volume, d'une part, et d'un nombre des voxels (13) voisins exclu du deuxième masque (MLV, 26') en volume, d'autre part.

**12.** Procédé suivant l'une des revendications précédentes, dans lequel on forme l'une ou les deux fonctions (29, 30) d'association, reposant sur le volume, respectivement, en fonction du deuxième masque (MLV) en volume, qui n'est pas adapté.

**13.** Installation de délimitation d'un objet (4) métallique dans un modèle (12) en volume formé à partir d'images (10) de projection d'un corps contenant l'objet (4) métallique, comportant un dispositif (7) de réception pour recevoir les images (10) de projection et un dispositif (12) de processeur, qui a des codes de programme conçus pour, lorsqu'il est exécuté par le dispositif (12) de processeur, effectuer un procédé suivant l'une des revendications précédentes.

FIG 1

EP 3 219 260 B1

FIG 2

FIG 3

$$LLHMetal(u,v) = -\frac{(P(u,v)-\mu_M)^2}{\sigma_M^2} - \frac{1}{2}\ln(\pi\sigma_M) + \alpha_M C(u,v) + \beta_M N_M(u,v) + \gamma_M M(u,v)$$

$$LLHBackground(u,v) = -\frac{(P(u,v)-\mu_B)^2}{\sigma_B^2} - \frac{1}{2}\ln(\pi\sigma_B) + \alpha_B C(u,v) + \beta_B N_B(u,v) + \gamma_B(1-M(u,v))$$

EP 3 219 260 B1

**FIG 4**

MLV

26'

$$\text{LLHMetal}(x,y,z) = -\frac{(V(x,y,z)-\mu_M)^2}{\sigma_M^2} - \frac{1}{2}\ln(\pi\sigma_M) + \beta_M N_M(x,y,z) + \gamma_M \text{MLV}(x,y,z)$$

29

30

$$\text{LLHBackground}(x,y,z) = -\frac{(V(x,y,z)-\mu_B)^2}{\sigma_B^2} - \frac{1}{2}\ln(\pi\sigma_B) + \beta_B N_B(x,y,z) + \gamma_B(1-\text{MLV}(x,y,z))$$

MLV

21'

26

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2015029178 A1

- DE 102011075912 A1

**In der Beschreibung aufgeführte Nicht-Patentliteratur**

- **W. J. H. VELDKAMP ; R. M. S. JOEMAI ; A. J. ; MOLEN, J. GELEIJNS.** Development and validation of segmentation and interpolation techniques in sinograms for metal artifact suppression in CT. *Medical Physics,* 2010, vol. 37, 620

- 3D Prior Image Constrained Projection Completion for X-ray CT Metal Artefact Reduction. **MEHRANIAN ABOLFAZL et al.** IEEE Transactions on Nuclear Science. IEEE Service Center, 01. Oktober 2013, vol. 60, 3318-3332

- **JIN, P. et al.** Joint Metal Artifact Reduction and Segmentation of CT Images using Dictionary-Based Image Prior and Continuously-Relaxed Potts Model. *Proceedings of the IEEE International Conference on Image Processing 2015,* 2015, 798-802